# EUROPEAN PATENT SPECIFICATION

(11) **EP 1 292 321 B1**
(45) Date of publication and mention of the grant of the patent: **26.10.2011**
(21) Application number: 01933328.5
(22) Date of filing: 11.05.2001
(51) Int. Cl.: A61K 39/00

(54) **METHOD TO INCREASE CLASS I PRESENTATION OF EXOGENOUS ANTIGENS BY HUMAN DENDRITIC CELLS**
VERFAHREN ZUR STEIGERUNG DER KLASSE I PRÄSENTATION VON EXOGENEN ANTIGENE DURCH HUMANE DENDRITISCHE ZELLEN
METHODE PERMETTANT D'AUGMENTER LA PRESENTATION DE CLASSE I D'ANTIGENES EXOGENES PAR LES CELLULES DENDRITIQUES HUMAINES

(30) Priority: 12.05.2000 US 203758 P
(43) Date of publication of application: 19.03.2003
(60) Divisional application: 10180857.4
(73) Proprietor: NorthWest Biotherapeutics, Inc., Bethesda, MD 20814 (US)
(72) Inventor: SALGALLER, Michael, L., Kirkland, WA 98034 (US); BOYNTON, Alton, L., Redmond, WA 98053 (US)
(74) Representative: Glawe, Delfs, Moll
(86) International application number: PCT/US2001/015428
(87) International publication number: WO 2001/087325

(56) References cited:
- WO-A-00/06723
- WO-A-01/00225
- WO-A-94/20127
- WO-A-97/04802
- US-A- 5 788 963
- HARRIS D T ET AL: "IMMUNOLOGIC APPROACHES TO THE TREATMENT OF PROSTATE CANCER" SEMINARS IN ONCOLOGY, BETHESDA, MD, US, vol. 26, no. 4, 1999, pages 439-447, XP000910090
- SALGALLER M L ET AL: "REPORT OF IMMUNE MONITORING OF PROSTATE CANCER PATIENTS UNDERGOING T-CELL THERAPY USING DENDRITIC CELLS PULSED WITH HLA-A2-SPECIFIC PEPTIDES FROM PROSTATE-SPECIFIC MEMBRANE ANTIGEN (PSMA)" PROSTATE, WILEY-LISS, NEW YORK, NY, US, vol. 35, no. 2, 1998, pages 144-151, XP000886069 ISSN: 0270-4137
- ZEOLI ET AL: "Expression of prostate specific molecules in BCG: A potential immunotherapeutic vaccine" PROCEEDINGS OF THE 91ST ANNUAL MEETING OF THE AMERICAN ASSOCIATION FOR CANCER RESEARCH. SAN FRANCISCO, CA, APRIL 1 - 5, 2000, PROCEEDINGS OF THE ANNUAL MEETING OF THE AMERICAN ASSOCIATION FOR CANCER RESEARCH, PHILADELPHIA, PA : AACR, US, vol. VOL. 41, 1 April 2000 (2000-04-01), page 875, XP002971593
- KAUFMANN STEFAN H E ET AL: "Impact of intracellular location of and antigen display by intracellular bacteria: Implications for vaccine development" IMMUNOLOGY LETTERS, vol. 65, no. 1-2, January 1999 (1999-01), pages 81-84, XP009045348 ISSN: 0165-2478
- STOVER C K ET AL: "NEW USE OF BCG FOR RECOMBINANT VACCINES" NATURE, MACMILLAN JOURNALS LTD. LONDON, GB, vol. 351, 6 June 1991 (1991-06-06), pages 456-460, XP002052118 ISSN: 0028-0836
- KLAGGE I ET AL: "Granulocyte-macrophage colony-stimulating factor elevates invariant chain expression in immature myelomonocytic cell lines" IMMUNOLOGY, vol. 91, no. 1, 1997, pages 114-120, XP002335272 ISSN: 0019-2805
- MURPHY ET AL.: 'Phase II prostate cancer vaccine trial: Report of a study involving 37 patients with disease recurrence following primary treatment' THE PROSTATE vol. 39, 1999, pages 54 - 59, XP002947177

## Description

### CROSS-REFERENCES TO RELATED APPLICATIONS

This application claims priority to U.S. Provisional Patent Application No. 60/203,758, filed May 12, 2000.

### BACKGROUND OF THE INVENTION

It is well established that the immune system can function to kill tumor cells, including both primary and metastatic cancer cells. Indeed, evidence that the immune system recognizes the presence of neoplastic cancerous cells is supported by the existence of infiltrating lymphocytes in tumor tissues (Haskill et al., Contemp. Top. Immunobiol. 8:107-170 (1978); Vose and Moore, Semin. Hematol. 22:27-40 (1985)). Despite the presence of immune cells, tumors often prevail and not only survive but metastasize to distant sites with unrestricted growth.

Recent advances in the understanding of T cell activation and recognition of target cells has permitted some progress in the development of T cell mediated cancer immunotherapy (Schwartz, Cell 71:1065-1068 (1992); Pardoll,, Curr. Opin. lmmunol. 4:619-623 (1992)).

The immune system develops from a single multi-potential progenitor cell into the major subgroups of lymphoid and myeloid cells. Lymphoid cells are comprised of B cells and T cells. Myeloid cells include macrophages, monocytes and neutrophils. Immune cells are capable of circulating and seeking out foreign antigens and eliminating them.

In the lymphoid subgroup, the immune response leads to the activation of helper T cells (T_{H}) which are positive for expression of the surface molecule CD4⁺ and of cytotoxic T cells (T_{C}) which are surface positive for the molecule CD8⁺. T cells are activated through interaction with antigen presenting cells (APC) that express major histocompatibility (MHC)-class I or -class II molecules associated with antigenic fragments. The associated antigenic amino acid sequences are specifically derived from the processed antigen.

APCs use two alternative methods to present antigens depending on the source of the antigen. Exogenous, soluble antigen is endocytosed into vacuoles and degraded by low pH. The peptide fragments that result are then directed to MHC-class II proteins and presented on the cell surface. Presentation on MHC-class I requires that antigens are degraded in the cytosol and transported by the TAP transporter system into the endoplasmic reticulum. Typically this requires that the antigen be in the cytosol, for example in the case of a viral infection or by cellular translation, and the resultant peptides then associate with MHC-class I.

The antigen-MHC complex is recognized by the specific T cell receptor which recognizes the antigen, and the CD4 and CD8 surface molecules. CD4 and CD8 interact with conserved regions of only one class of MHC each. Whereas MHC-class II is recognized by T_{H} cells due to interaction with CD4, MHC-class I presentation is restricted to activating T_{C} cells through interaction with CD8.

The activation of naive or primed T cells follows a defined mechanism. Endogenous antigen is presented on MHC-class I and soluble exogenous antigen is presented on MHC-class II by APCs. The MHC-class I or MHC-class II antigen complexes interact with CD8 or CD4 respectively and also interact with the T cell receptor specific for the antigen. Upon this specific interaction, secondary molecules such as β-microglobin and CD28 trigger activation of the T cells which then exert the appropriate immune response.

The sensitized or "primed" CD4⁺ T cells produce chemokines that participate in the activation and recruitment of B cells as well as various T cell subsets. The sensitized CD8⁺ T cells increase in numbers in response to lymphokines and are capable of destroying any cells that express the specific antigenic fragments associated with matching MHC-class I molecules (Jondal et al., Immunity 5:295-302 (1996)).

Tumor infiltrating lymphocytes are evidence that cancerous tumors induce CD8⁺ CTL capable of eradicating cells expressing cancer associated or cancer specific antigens, thereby limiting the progression of tumor spread and disease development However, tumors frequently grow and metastasize, overcoming this natural immunity. Various methods for immunotherapy directed to a number of particular cancers have been suggested to enhance this natural immune response, however, the primary difficulty has been inducing APCs to present soluble human tumor associated or tissue specific antigens via MHC-class I. Recent experiments have demonstrated in murine systems, that activation of CTLs *in vitro* can confer a potent protection from growth of syngeneic tumors *in vivo* (Fields et al., Proc. Natl. Acad. Sci. USA 95:9482-9487 (1998)). However, experiments in the murine immune system are not completely predictive of human immune responses. To date there are no therapeutic methods that successfully elicits an effective human CTL immunotherapeutic response against primary or metastatic cancer using APCs incubated with a soluble protein or proteinaceous antigen.

Antigen presenting cells (APCs) are particularly important in eliciting an effective immune response. By definition, APCs not only present antigens to T cells with antigen-specific T cell receptors, but provide all the signals necessary for T cell activation. The signals necessary for T cell activation are incompletely defined, but probably involve a variety of cell surface molecules as well as cytokines or growth factors. Further, the factors necessary for the activation of naive or unprimed T cells may be different from those required for the re-activation of previously primed memory T cells. The ability of APCs to both present antigens and deliver signals for T cell activation is commonly referred to as an accessory cell function. Although monocytes and B cells have been shown to be competent APC, their antigen presenting capacities *in vitro* appear to be limited to the re-activation of previously sensitized T cells. Hence, they are not capable of directly activating functionally naive or unprimed T cell populations.

The term "dendritic cells" refers to a diverse population of morphologically similar cell types found in a variety of lymphoid and non-lymphoid tissues (Steinman, Ann. Rev. Immunol. 9:271-296 (1991)). These cells include lymphoid dendritic cells of the spleen, Langerhans cells of the epidermis, and veiled cells in the blood circulation. Although they are collectively classified as a group based on their morphology, high levels of surface MHC class II expression, and absence of certain other surface markers expressed on T cells, B cells, monocytes, and natural killer cells, it is presently not known whether dendritic cells derive from a common precursor or can all function as APCs in the same manner. Dendritic cells are the most potent APCs of the immune system capable of stimulating primary T and B lymphocyte responses. (Banchereau et al., Nature 392:245-252 (1998)).

Studies have described methods for the isolation and expansion of human DCs from many sources, including, from human peripheral blood. (Macatonia et al., Immunology 74:399-406 (1991); O'Doherty et al., J. Exp. Med. 178:1067-1078 (1993) (isolation); and Markowicz et al., J. Clin. Invest. 85:955-961 (1991); Romani et al., J. Exp. Med 180:83-93 (1994); Sallusto et al., J. Exp. Med. 179:1109-1118(1994); Bernhard-et al., Cancer Res. 55:1099-1104 (1995) (expansion)). PCT Publication WO 94/02156 describes a method for isolating human DCs to present antigens to induce antigen specific T cell-mediated responses. Adoptive cellular immunotherapy and use of the isolated DCs against infectious diseases and cancer are mentioned.

Clinical trials have been initiated relating to the use of dendritic cells pulsed with a relevant antigen against melanoma (Nestle, F.O., et al., Nat. Med. 4:328-332 (1998)); against B cell lymphoma (Hsu, F.J., et al., Nat. Med. 2:52-58 (1996)); and against prostate cancer (U.S. Patent No. 5,788,963 to Murphy et al; Murphy et al., Prostate 29:371-380 (1996); Salgaller, et al., Prostate 35:144-151 (1998)).

Exogenous antigen processing results in antigen presentation on MHC-class II, whereas the endogenous processing pathway utilizes MHC-class I (Jondal et al., Immunity, 5:295-302 (1996)). The activation of T_{H} cells by the MHC-class II presentation of antigen has had therapeutic success, but experiments in murine models suggest a significantly more potent cancer protection would result if presentation were by MHC-class I. However, success in a murine model is not always predictive of success using human cells and there have been no reports of MHC-class I presentation of soluble exogenous antigens by human APCs.

Although there is a small subgroup of soluble antigens that are presented on MHC-class I, including some bacterial and viral antigens, a method of reliably inducing MHC-class I presentation of many antigens, including, for example, exogenous soluble human tumor associated or tissue specific antigens has not been reported. Techniques have been developed to create fusion proteins of soluble antigens that are presented on MHC-class I and antigens of interest, however this process requires significant time and molecular manipulations to implement effectively. MHC-class I processing of an exogenous antigen could potentially represent a significant improvement in current immunotherapies.

Prostate cancer is the most common form of cancer currently diagnosed in American men. It is second only to lung cancer as the leading cause of cancer deaths among adult males. Nearly a third of all newly diagnosed prostate cancer patients present with metastatic or locally advanced disease. At present, available therapies for metastatic disease, including hormonal, chemotherapeutic and radiation approaches, have not achieved curative potential in a significant percentage of patients. For those with localized carcinoma, prostatectomy and radiotherapy, the current standards of treatment result in failure rates of between 20 and 50%. The options for these primary treatment failures, as with those with progressed disease, are few in number and limited in clinical benefit.

With the elucidation of the mechanisms involved in immune recognition, new and exciting strategies in anti-cancer therapeutics have become available. Of particular promise are those cancer vaccines which utilize antigen presentation by dendritic cells (DC)s to elevate the anti-tumor response. However, recent work has shown that it is important to enhance the delivery of soluble, exogenous, tumor-associated antigens into the major histocompatibility complex (MHC) class I processing compartment of DCs to maximize the cellular immune response. The present invention addresses these and other needs in the art

### SUMMARY OF THE INVENTION

The present invention is defined in the claims and provides methods for dendritic cell activation of T cells in immunotherapeutic responses against primary or metastatic cancer. The DCs obtained from human donors are administered to a cancer patient in need thereof, following exposure to a soluble tissue associated, tissue specific, tumor associated, or tumor specific antigen in combination with an adjuvant that increases the MHC class-I associated cytotoxic T cell response *in vivo* as compared to the response induced by antigen alone. Alternatively, the antigen used for exposure to the DCs can be a fragment of the tissue associated, tissue specific, or tumor associated antigen. The DCs can be exposed simultaneously to the adjuvant and the soluble tissue associated, tissue-specific or tumor antigen, or antigenic fragments thereof. This response includes helper T cell (T_{H}) and cytotoxic T cell (Tc) activation. Alternatively, human T cells are cultured *in vitro* with the foregoing DCs and the *in vitro* activated T cells are subsequently administered to a cancer patient in need thereof.

In the invention as claimed in the claims, bacillus Calmette Guerin (BCG) *Mycobacteria bovis*, is used with an tumor cell antigen to obtain MHC-class I processing. Exogenous antigen is normally processed by the MHC-class II compartment in antigen presenting cells (APC) and endogenous antigens are processed by the MHC-class I compartment. Surprisingly, the present inventors have found that when DCs are pulsed with a soluble antigen, including human tumor antigen or tissue specific antigens with an adjuvant such as BCG, enhancement of MHC-class I presentation occurs. Therefore, the presence of an adjuvant such as BCG typically increases DC soluble tumor antigen processing in the MHC-class I compartment and correspondingly, activates a higher percentage of CD8⁺ T cells when compared to individuals administered the antigen alone.

DCs can be exposed to soluble antigen, including viral, bacterial, tissue, tissue specific, tumor associated, or tumor specific antigen in the presence of a combination of BCG and a bacterial exotoxin, such as, lipopolysaccharide (LPS). According to one embodiment a prostate tumor cell lysate recovered from a surgical specimen can be used as antigen. For example, a sample of a prostate cancer patient's own tumor, obtained at biopsy or at surgical resection, can be used to provide a cell lysate for antigen. Further, purified prostate specific membrane antigen (PSMA, also known as PSM antigen), which specifically reacts with monoclonal antibody 7E11-C.5 can be used as an antigen. Additional antigens include antigenic fragments of a tissue associated, tissue specific, tumor associated or tumor specific protein antigen, *i.e.*, such as PSMA, prostate mucin antigen, prostate specific antigen, prostate acid phosphatase (PAP), PD41 antigen, and the like. According to one embodiment an antigenic peptide having the amino acid sequence Leu Leu His Glu Thr Asp Ser Ala Val (SEQ ID NO: 1)(designated PSM-1) which corresponds to amino acid residues 4-12 of PSMA can be used as antigen. Additionally, an antigenic peptide having an amino acid sequence Ala Leu Phe Asp Ile Glu Ser Lys Val (SEQ ID NO: 2) (designated PSM-2), which corresponds to amino acid residues 711-719 of PSMA can be used as antigen.

An antigenic peptide selected from antigenic peptide fragments of PSM can be matched to a binding motif of a specific haplotype. The peptides can be selected to be presented by DCs to activate T cells of a patient which matched the haplotype indicated for each peptide of PSA and which have been matched to a binding motif of a specific haplotype.

The MHC class-I antigen loaded DCs, described *supra*, can be incubated *in vitro* with primed or unprimed T cells to activate the relevant T cell responses. The activated T cells can be subsequently administered to a patient, i.e., a cancer for immunotherapy. In either case, the DCs are advantageously used to elicit an immunotherapeutic growth inhibiting response against, for example, an infection or a primary or metastatic human cancer. In particular, the human cancer is prostate cancer.

The invention as defined in the claims is useful in a method for producing a tumor cell proliferation growth inhibiting response, which comprises administering, to a cancer patient in need thereof, an effective amount of activated T cells, in which the T cells were activated *in vitro.* The *in vitro* activation includes exposure of human dendritic cells to a tissue associated, tumor associated, tumor specific antigen or antigenic fragments thereof in combination with BCG, either in combination with or without LPS, to enhance MHC-class I processing. The invention relates to a method for producing a tumor growth or cancer cell proliferation inhibiting response, which comprises administering, to a cancer patient in need thereof, an effective amount of human dendritic cells, exposed *in vitro* to a tissue associated, tissue specific, tumor associated or tumor specific antigen or an antigenic fragment thereof in combination with BCG, in combination with or without LPS, such that after administration, the human DCs elicit a predominately CD8⁺ T cell immune response or augment an existing immune response against the tumor or cancer cells.

Antigens useful for the methods of the invention include but are not limited to; soluble extracts of tumor cells from a patient biopsy, soluble extracts from tumor cells obtained during surgical resection, tumor specific antigens, tissue associated or tissue specific antigens relevant to the tumor type, recombinant purified tumor antigens, recombinant purified tissue associated or tissue specific antigens, and the like, as set forth herein.

### BRIEF DESCRIPTION OF THE FIGURES

The present invention may be more fully understood by reference to the following detailed description of the invention, illustrated examples of specific embodiments of the invention and the appended figures.

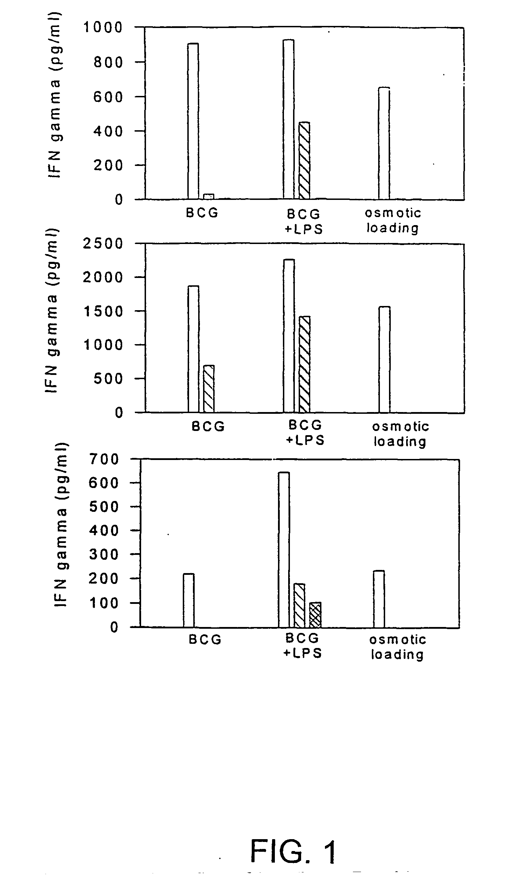

Fig. 1A through Fig. 1C depict the activation of T cells from prostate cancer patients by autologous (Fig. 1A) or allogeneic (Fig. 1B and Fig. 1C) dendritic cells previously loaded by pulsing with LNCaP-derived prostate specific membrane antigen (PSMA) and either BCG, or BCG and LPS, or the T cells were pulsed with dendritic cells osmotically loaded with PSMA alone. Day 18 cultured T cells from Patient 92 were washed and added to 96-well plates at 5x10⁴ cells per well in duplicate. Autologous DCs (Fig. 1A), or allogeneic DCs from Patient 105 (Fig. 1B) and patient LT. (Fig. 1C), osmotically loaded with PSMA (open bars) or ovalbium (OVA; hatched bars) (or left untreated; crossed bars) were added to the T cells at 5x10⁴ cells per well to test for activation as measured by cytokine production. After 24 hours incubation, 150 µl of supernatant was removed from each culture well and the amount of IFNγ present measured by ELISA and plotted against the y-axis.

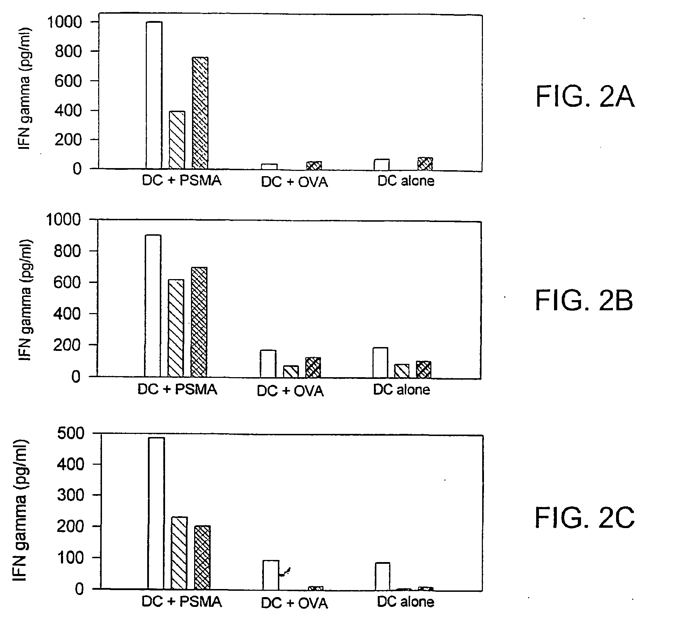

Fig. 2A through Fig. 2C depict the specific reactivity of T cells activated *in vitro*, including both CD8⁺ and CD4⁺ T cell groups. Day 25 cultured T cells from Patient 105 were washed and added to 96-well plates at 5 x10⁴ cells per well in duplicate. DCs were pulsed with antigen (PSMA or OVA) and either (Fig. 2A) BCG, (Fig. 2B) BCG + LPS. Alternatively, PSMA or OVA were osmotically loaded (Fig. 2C). Autologous DCs pulsed with PSMA (DC+PSMA), OVA (DC+OVA), or unpulsed (DC alone) were added to Patient 105 T cells at 5 x 10⁴ DCs per well. Effector cells were incubated with either saline (No mAb; open bars), or 1 µg/ml anti-CD8 mAb (hatched bars), or 1 µg/ml anti-CD4 mAb (crossed bars), in duplicate wells. IFNγ production was measured as in Fig. 1.

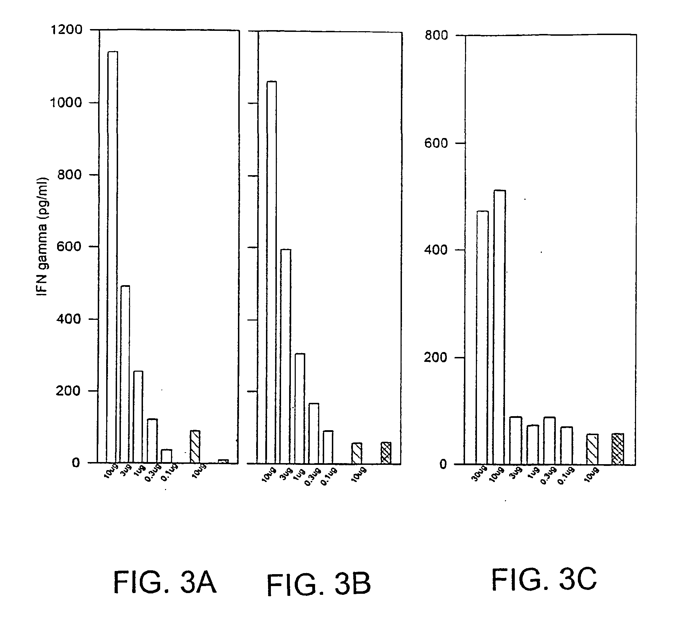

Fig. 3A through Fig. 3C depict dose dependent effects of dendritic cells activated *in vitro* with soluble PSMA combined with BCG, or with BCG and LPS on T cells. T cells from prostate cancer Patient 105 were activated by autologous dendritic cells previously loaded with serial dilutions of PSMA derived from LNCaP cells. ELISAs were performed to assess IFNγ secretion. Day 32 or 39 (Fig. 3C) cultured cells were washed and added to 96-well plates at 5 x10⁴ cells per well in duplicate. Autologous DC pulsed with either PSMA (open bars), OVA (hatched bars), or unpulsed (crossed bars) were added to the T cells at 5 x 10⁴ cells per well with or without BCG or BCG plus LPS. BCG, day 32 of culture (Fig. 3A); BCG + LPS, day 32 of culture (Fig. 3B); BCG, day 39 of culture (Fig. 3C). After 24 hours incubation, 150 µl of supernatant was removed from each culture and the amount of IFNγ present was measured by ELISA.

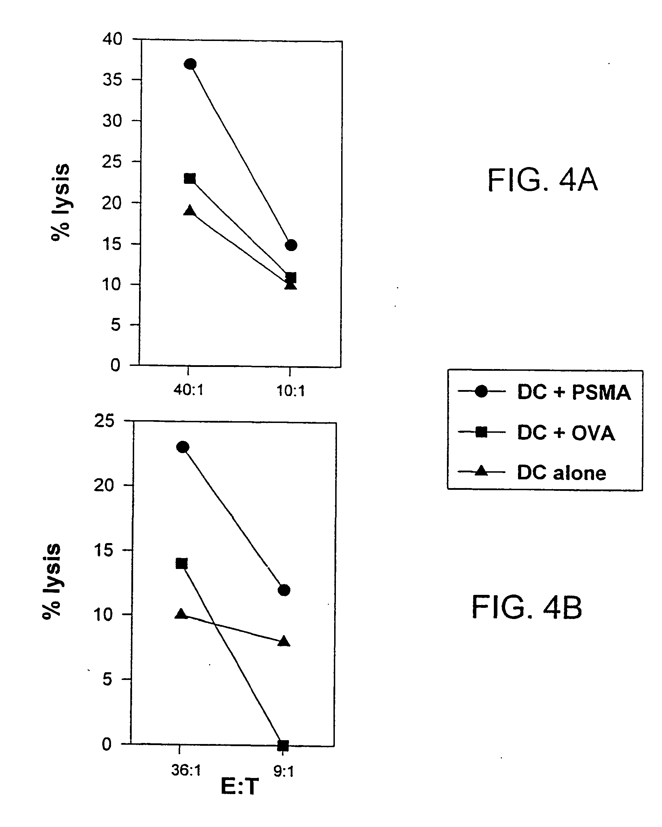

Fig. 4A and Fig. 4B demonstrate the stimulation of lytic activity for antigen-specific targets of T cells from prostate cancer patients stimulated *in vitro* with PSMA-expressing DCs. Different ratios of effector (E) (i.e., T cells) to target M(ie., autologous dendritic cells) (E:T) were incubated for four hours. The autologous DCs, osmotically loaded with PSMA (●) or OVA (■), or untreated (▲), were radiolabeled with ¹¹¹In. Percent lysis was calculated using the following formula: [(experimental release - spontaneous release)/(maximum release - spontaneous release)] x 100. Patient I.T., day 32 of culture (Fig. 4A). Patient 92, day 39 of culture (Fig. 4B).

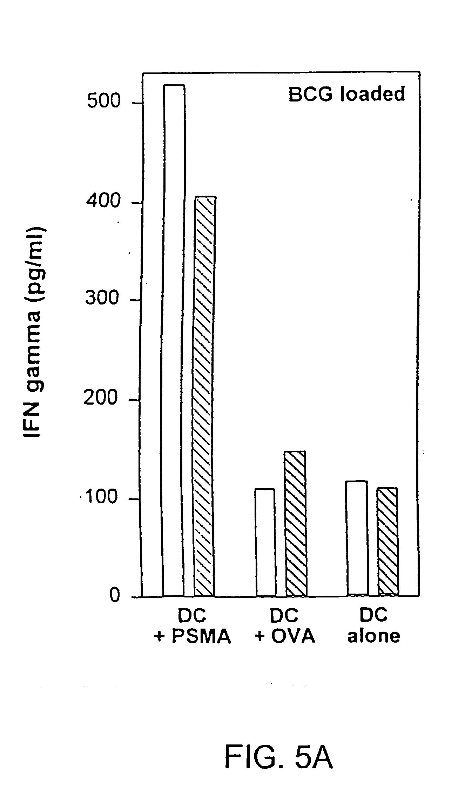

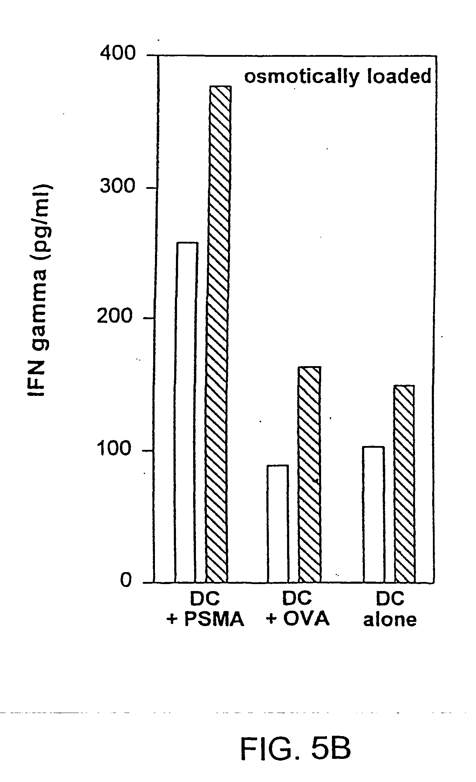

Fig. 5A and Fig. 5B depicts PSMA specific reactivity of T cells from prostate cancer Patient 105 activated by either fresh or cryopreserved autologous DCs, loaded with PSMA partially purified from LNCaP cells (approximately 80% pure). Day 39 cultured T cells were washed and added to 96-well plates at 5 x10⁴ cells per well in duplicate. Autologous DC targets were pulsed with PSMA, OVA, or unpulsed and were added to the T cells at 5 x10⁴ cells per well. PSMA specific reactivity was observed against both fresh (open bars) and cryopreserved (hatched bars) DC targets. PSMA specific reactivity occurred whether the effectors had been stimulated with osmotically loaded or BCG loaded DCs. IFNγ production was measured as in Fig. 1.

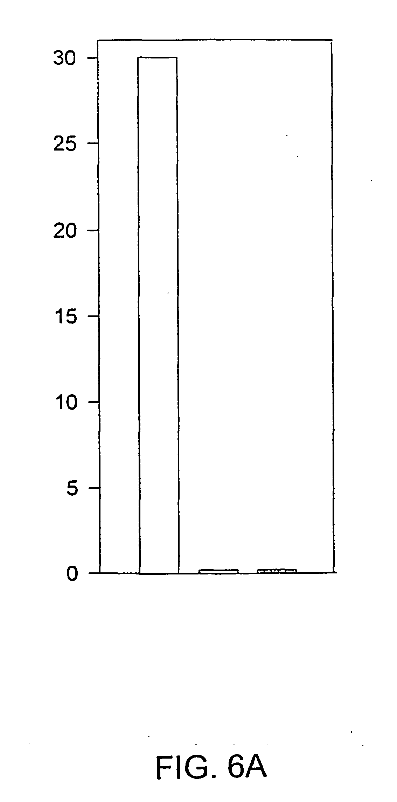

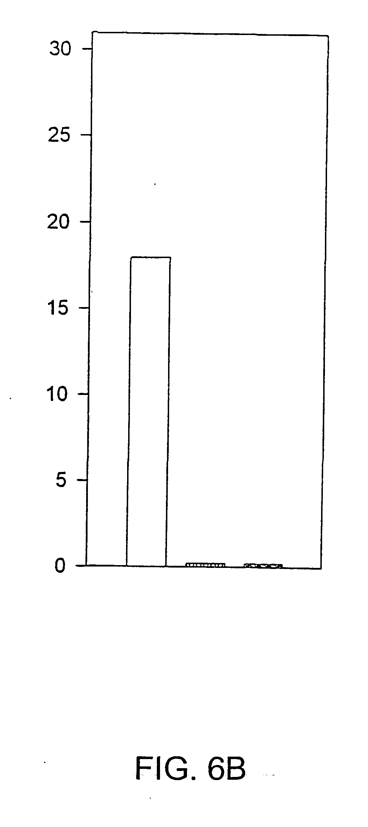

Fig. 6 demonstrates that T cells from prostate cancer Patient 92 can be activated by the antigen presenting cell line, T2, exogenously loaded with 25 µg peptide either with PSM-P1 (open bars), the influenza matrix protein M1 (hatched bars), or nothing (crossed bars). Standard ELISAs were performed to assess IFNγ secretion. Day 46 of culture (Fig. 6A) or Day 53 of culture (Fig. 6B).

### DETAILED DESCRIPTION OF THE INVENTION

The present invention is defined in the claims and relates to methods, and compositions, for use of dendritic cells (DCs) to activate T cells for an immunotherapeutic response against an antigen. The antigen can be any antigen, including a viral or bacterial antigen, a tissue antigen, a tumor associated antigen or other antigen associated with, for example, a primary or metastatic cancer. The DCs obtained from human donors are administered to a patient to activate the relevant T cell responses *in vivo* subsequent to exposure to a virus, a bacteria or to a tissue associated, a tissue specific, a tumor or cancer associated, or tumor specific antigen in combination with a factor or agent that promotes Major Histocompatibility Complex-(MHC) class-I processing. The antigen can be a fragment of one of the antigens provided above. Further, and optionally the methods and compositions induce DC maturation. The factor or agent thus serving to promote DC activation of T cells such that at least 25%, and even over 50% of the T cells, as compared to antigen provided alone, are CD8⁺. Alternatively, the DCs are exposed to a tissue specific antigen, a cancer antigen or an antigenic fragment of either antigen with the factor promoting MHC-class I processing and maturation of DCs *in vitro* and subsequently incubated with primed or unprimed T cells to activate the relevant T cell responses *in vitro.* The activated T cells are then administered to a cancer patient in need thereof. In either case, the DCs are advantageously used to elicit an immunotherapeutic growth inhibiting response against a primary or metastatic cancer tumor.

Solely for ease of explanation, the description of the invention is divided into the following sections: (1) sources of antigen, (2) methods for obtaining or isolating dendritic cells, including DCs with extended lifespan or cryopreserved DCs; and (3) applications or methods of use of DCs to stimulate cytotoxic and helper T cells against a virus, bacteria or cancer *in vitro* and *in vivo*.

Antigen-reactive T cells are antigen-specific effector cells that are important in resisting disease, including cancer. Antigen-reactive T cells which are CD8⁺, recognize antigen presented by MHC-class I molecules. MHC-class I molecules are expressed by almost all cell types. Antigen-reactive T cells which are CD4⁺, recognize antigen presented by MHC-class II molecules. MHC-class II molecules are expressed in a variety of cell types including dendritic cells, endothelial cells, monocytes, macrophages, and lymphocytes. The ability of antigen-reactive T cells to kill target cells is restricted by antigenic and genetic factors. For lysis of target cells, the target cells must carry the same antigen that originally induced the stimulation of the T cells, and the same class MHC molecule as the T cells.

The present invention is defined in the claims and relates to methods of generating T cells reactive to an antigen that can be used in the prevention or treatment of a disease or disorder, such as a viral or bacterial infection, or cancer. This invention was made possible by the surprising discovery that bacillus Calmette Guerin (BCG) stimulates MHC-class I processing of exogenous soluble antigen and subsequently increases preferential activation of CD8⁺ T cells to at least 25%, and even greater than 50% of the activated T cell population, when compared to individuals administered antigen alone. The proportion of CD8⁺ T cells can increase to 25%, 50%, or more, and can be even greater than 75% of the total T cell response.

BCG with a viral, a bacterial, a tissue associated, tissue specific, tumor associated or tumor specific antigen, or an antigenic fragment thereof can be added multiple times to the *in vitro* cultures in order to restimulate antigen-reactive T-cell proliferation. The antigen-reactive T cells generated by the methods of the invention are capable of specifically targeting, killing, or causing lysis of the infected cells or cancer cells, or other target cells as the case may be, or any cells bearing the same antigens and similar MHC molecules with which the T cells are prepared. The antigen-reactive T cells of the invention can also secrete one or more measurable cytokines, such as IL-2, IFN-γ, TNFβ, IL-4, IL-5, IL-6, IL-9, IL-10, IL-3, and/or GM-CSF. The production of these cytokines can be used to monitor specific T cell activation *in vitro.*

Previously, BCG has been used as a constituent of various vaccine compositions to act as an adjuvant for augmenting a serological or antibody immune response to the target immunogen. Further, dendritic cells have been shown to internalize particles, including BCG mycobacteria. (Inaba, et al., J. Exp. Med. 178:479-488 (1993)). The mycobacteria-laden dendritic cells have been shown to be more potent in presenting antigens to primed T cells then corresponding cultures of mature dendritic cells that are exposed to a pulse of BCG. (Inaba et al., *supra* (1993)).

Dendritic cell activation by BCG has been characterized as involving homotypic aggregation, up regulation of surface antigens, down modulation of endocytic activity and the release of tumor necrosis factor-α. (Thurnher, et al., Int. J. Cancer 70:128-134 (1997)). Enhanced expression has been documented for dendritic-cell-maturation antigen CD83 and of the T cell co-stimulator CD86 (B7-2). It has been suggested that induction of secretion of TNF-α was at least in part responsible for the observed phenotypic and functional changes observed in dendritic cells following uptake of BCG. Stimulation of IL-8 mRNA expression and IL-8 protein secretion has also been associated with T cell effects of BCG. (Ramoner et al., J. Urology 159:1488-1492 (1998)).

To date, although BCG has been administered in conjunction with various antigens, including cancer cells and cancer associated antigens, there has apparently been no demonstration or recognition of a preferential activation of CD8⁺ T cells when exposed to dendritic cells that have been activated with tissue specific antigen combined with BCG, or with BCG and LPS inducing a MHC-class I response.

The antigen-reactive T cells can be administered *in vivo* autologously (i.e., to the same individual from which the T cells (or parental cells to the T cells) were originally obtained) or sygeneically (i.e., to an identical twin of the individual from which the cancer or infected cells were initially obtained); or allogeneically to an individual who shares at least one common MHC allele with the individual from which the antigenic cells and T cells were originally obtained.

As used herein, the term "antigenic cells" refers to any cells, typically infected cells or cancer cells, and in particular, prostate cancer cells, which can elicit an immune response in a subject. The sources of antigenic cells, and methods of preparation of antigenic cells for use in the present methods are discussed in this section.

The term "pulsed" as used herein includes the process of immunization in *vitro.* The process of immunization *in vitro* can be performed by a variety of methods including but not limited to the dendritic cells pulsed with antigens (Steel and Nutman, J. Immunol. 160:351-360 (1998); Tao et al., J. Immunol. 158:4237-4244 (1997); Dozmorov and Miller, Cell Immunol. 178:187-196 (1997); Inaba et al., J Exp Med. 166:182-194 (1987); Macatonia et al., J Exp Med. 169:1255-1264 (1989); De Bruijn et al., Eur. J. Immunol. 22:3013-3020 (1992)), RMA-S cells (mutant cells expressing high numbers of 'empty' cell surface class I MHC molecules) loaded with peptide (De Bruijn et al., Eur. J. Immunol. 21:2963-2970 (1991); De Bruijn et al., Eur. J. Immunol. 22:3013-3020 (1992); Houbiers et al., Eur. J. Immunol. 26:2072-2077 (1993)) and macrophage phagocytosed-peptide loaded beads (De Bruijn et al., Eur. J. Immunol. 25:1274-1285 (1995)), and osmotically stressed antigenic cells (PCT publication WO 98/15616)). Priming, therefore, results in the first exposure of naive immune cells to an antigenic molecule.

The term "pulsing" as used herein refers to the process of exposing primed immune cells *in vitro* to BCG, and alternatively BCG and LPS, and a viral antigen, bacterial antigen, tissue specific antigen, a tumor antigen, or an antigenic fragment of the antigen. The BCG and viral antigen, bacterial antigen, tissue specific or tumor associated antigens, as used herein, comprises a non-covalent mixture of BCG and an antigenic molecule.

The term "antigen," and "antigenic molecule" as used herein comprises viral, bacterial, tissue associated or tissue specific and tumor associated or tumor specific protein antigens useful for presentation by the dendritic cells to activate T cells for immunotherapeutics. In particular, for developing an immune response to the infecting virus or bacteria or to prostate cells or prostate associated antigens, i.e., PSMA in prostatic tumor vasculature.

According to one embodiment, a prostate tumor cell lysate recovered from surgical specimens can be used as antigen. For example, a sample of a cancer patient's own tumor, obtained at biopsy or at surgical resection, can be used to provide a cell lysate for antigen. Alternatively, a membrane preparation of tumor cells of a prostate cancer patient can be used.

According to yet another embodiment, purified prostate specific membrane antigen (PSMA, also known as PSM antigen), which specifically reacts with monoclonal antibody 7E11-C.5 (*see* generally Horoszewicz et al., Prog. Clin. BioL Res. 37:115-132 (1983), US Patent No. 5,162,504, US Patent-No. 5,788,963, Feng et al., Proc. Am. Assoc. Cancer Res. 32:(Abs. 1418)238 (1991)) can be used as antigen. Cloning of the gene encoding the PSMA antigen has been described by Israeli et al., Cancer Res. 54:1807-1811. Expression of the cloned gene, *e.g.,* in yeast cells, can provide a ready source of the PSMA antigen for use according to the present invention.

In still yet another embodiment, an antigenic peptide having the amino acid residue sequence Leu Leu His Glu Thr Asp Ser Ala Val (SEQ ID NO: 1)(designated PSM-P1) which corresponds to amino acid residues 4-12 of PSMA can be used as antigen. According to another embodiment, an antigenic peptide having the amino acid residue sequence Ala Leu Phe Asp Ile Glu Ser Lys Val (SEQ ID NO: 2) (designated PSM-P2) which corresponds to amino acid residues 711-719 of PSMA can be used as antigen.

According to another embodiment, an antigenic peptide having an amino acid residue sequence Xaa Leu (or Met) Xaa Xaa Xaa Xaa Xaa Xaa Val (or Leu) (designated PSM-PX) where Xaa represents any amino acid residue can be used as antigen. This peptide resembles the HLA-A0201 binding motif, *i.e.*, a binding motif of 9-10 amino acid residues with "anchor residues", leucine (Leu) and valine (Val) found in HLA-A2 patients (Grey et al., Cancer Surveys 22:37-49 (1995)). This peptide is typically used as antigen for HLA-A2⁺ patients. (*see*, Central Data Analysis Committee 'Allele Frequencies ", Section 6.3, Tsuji, K. et al., (eds.), Tokyo University Press, pp. 1066-1077).

In yet another embodiment, an antigenic peptide selected from the peptides listed in Table 1A (below) can be used as antigen. The peptides listed in Table 1A have amino acid residue sequences corresponding to fragments of PSM and have been matched to a binding motif of a specific haplotype. According to one embodiment, the peptides are selected to be presented by dendritic cells to activate T cells of a patient which matched the haplotype indicated for each peptide in Table 1A.

**Table 1A**

| **PSM Peptides*** | | | | |
|---|---|---|---|---|
| **ID No.** | **Haplotype** | **Initial Amino Acid Residue**** | **Amino Acid Sequence** | **SEQ ID NO:** |
| PSM-P3 | A2 | 20 | Trp Leu Lys Ala Gly Ala Leu Val Leu | 3 |
| PSM-P4 | A2 | 27 | Val Leu Ala Gly Gly Phe Phe Leu Leu | 4 |
| PSM-P5 | A2 | 109 | Glu Leu Ala His Tyr Asp Val Leu Leu | 5 |
| PSM-P6 | A2 | 260 | Asn Leu Asn Gly Ala Gly Asp Pro Leu | 6 |
| PSM-P7 | A2 | 461 | Thr Leu Arg Val Asp Cys Thr Pro Leu | 7 |
| PSM-P8 | A2 | 660 | Val Leu Arg Met Met Asn Asp Gln Leu | 8 |
| PSM-P9 | A2 | 568 | Pro Met Phe Lys Tyr His Leu Thr Val | 9 |
| PSM-P10 | A2 | 57 | Asn Met Lys Ala Phe Leu Asp Glu Leu | 10 |
| PSM-P11 | A2 | 469 | Leu Met Tyr Ser Leu Val His Asn Leu | 11 |
| PSM-P12 | A2 | 663 | Met Met Asn Asp Gln Leu Met Phe Leu | 12 |
| PSM-P 16 | A1 | 171 | Glu Gly Asp Leu Val Tyr Val Asn Tyr | 13 |
| PSM-P17 | A1 | 264 | Ala Gly Asp Pro Leu Thr Pro Gly Tyr | 14 |
| PSM-P18 | A1 | 463 | Arg Val Asp Cys Thr Pro Leu Met Tyr | 15 |
| PSM-P19 | A1 | 143 | Leu Phe Glu Pro Pro Pro Pro Gly Tyr | 16 |
| PSM-P20 | A1 | 558 | Thr Tyr Glu Leu Val Glu Lys Phe Tyr | 17 |
| PSM-P21 | A1 | 701 | Ala Gly Glu Ser Phe Pro Gly Ile Tyr | 18 |
| PSM-P22 | A1 | 725 | Trp Gly Glu Val Lys Arg Gln Ila Tyr | 19 |
| PSM-P26 | A11 | 398 | Ile Val Arg Ser Phe Gly Thr Leu Lys Lys Glu | 20 |
| PSM-P27 | A11 | 63 | Asp Glu Leu Lys Ala Glu Asn Ile Lys Lys Phe | 21 |
| PSM-P28 | A11 | 491 | Lys Ser Leu Tyr Glu Ser Trp Thr Lys Lys Ser | 22 |
| PSM-P36 | A24 | 448 | Ala Tyr Ile Asn Ala Asp Ser Ser Ile | 23 |
| PSM-P37 | A24 | 606 | Lys Tyr Ala Asp Lys Ile Tyr Ser Ile | 24 |
| PSM-P38 | A24 | 298 | Gly Tyr Tyr Asp Ala Gin Lys Leu Leu | 25 |
| PSM-P39 | A24 | 624 | Thr Tyr Ser Val Ser Phe Asp Ser Leu | 26 |
| PSM-P40 | A24 | 178 | Asn Tyr Ala Arg Thr Glu Asp Phe Phe | 27 |
| PSM-P41 | A24 | 227 | Leu Tyr Ser Asp Pro Ala Asp Tyr Phe | 28 |
| PSM-P46 | B3501 | 289 | Leu Pro Ser Ile Pro Val His Pro Ile | 29 |
| PSM-P47 | B3501 | 501 | Ser Pro Ser Pro Glu Phe Ser Gly Met | 30 |

| | | | | |
|---|---|---|---|---|
| * "PSM peptides" refers to peptides having an amino acid sequence corresponding to a fragment of PSMA (a/k/a PSM). ** "Initial Amino Acid Residue" refers to the residue number of the amino acid of PSM to which the first amino acid of the peptide corresponds. | | | | |

In another embodiment of the present invention, prostate specific antigen (PSA) (*see*, Pepsidero et al., Cancer Res. 40:2428-2432 (1980); McCormack et al., Urology 45:729-744 (1995)) can be used as antigen.

According to another embodiment, an antigenic peptide selected from the peptides listed in Table 1B (below) can be used as antigen. The peptides in Table 1B have amino acid residue sequences corresponding to fragments of PSA and have been matched to a binding motif of a specific haplotype as indicated in Table 1B. According to one embodiment, the peptide is presented by dendritic cells to activate T cells of patients which match the haplotype indicated for each peptide in Table 1B.

**Table 1B**

| **PSA Peptides*** | | | | |
|---|---|---|---|---|
| **Iden. No.** | **Haplotype** | **Initial Amino Acid Residue**** | **Amino Acid Sequence** | **SEQ ID NO.** |
| PSA-P1 | A2 | 53 | Val Leu Val His Pro Gln Trp Val Leu | 31 |
| PSA-P2 | A2 | 171 | Lys Leu Gln Cys Val Asp Leu His Val | 32 |
| PSA-P11 | A1 | 235 | Ala Leu Pro Glu Arg Pro Ser Leu Tyr | 33 |
| PSA-P21 | A11 | 25 | Ile Val Gly Gly Trp Glu Cys Glu Lys | 34 |
| PSA-P22 | A11 | 185 | Gln Val His Pro Gln Lys Val Thr Lys | 35 |
| PSA-P23 | A11 | 245 | Val Val His Tyr Arg Lys Trp Ile Lys | 36 |
| PSA-P31 | A24 | 152 | Cys Tyr Ala Ser Gly Trp Gly Ser Ile | 37 |

| | | | | |
|---|---|---|---|---|
| * "PSA peptides" refers to peptides having an amino acid sequence corresponding to a fragment of PSA. ** "Initial Amino Acid Residue" refers to the residue number of the amino acid of PSA to which the first amino of the peptide corresponds. | | | | |

According to still another embodiment, a prostate mucin antigen, recognized by monoclonal antibody PD41, described by Wright (US Patent No. 5,227,471 and No. 5,314,996; Beckett et al. Cancer Res. 51:1326-1222 (1991)) can be used as antigen. Alternatively, a crude lysate of prostate tumor cells comprising antigen which binds to the antibody produced by the hybridoma cell line ATCC HB 11094 and which express the PD41 mucin antigen can be used as antigen.

Additional prostate antigens which can be used in the methods of the present invention include, but are not limited to, six transmembrane epithelial antigen of the prostate (STEAP; Hubert et al., Proc. Natl. Acad. Sci. USA 96:14523-14528 (1999)), prostate carcinoma tumor antigen (PCTA-1; Su et al. Proc. Natl. Acad. Sci. USA 93:7252-7257 (1996)); prostate stem cell antigen (PSCA; Reiter, et al., Proc. Natl. Acad. Sci. USA 95:1735-1740 (1998)). Antigenic fragments of each antigen are also considered to be encompassed by the scope of the present invention.

Additional antigens include, but are not limited to, viral neutralization antigens or antigenic peptides. Further, bacterial proteins, glycoproteins, glycolipids or carbohydrates and antigenic fragments thereof, are considered part of the present invention.

According to the present invention, cellular immunotherapy is developed by obtaining antigenic cells and immune cells from one or more individuals, more typically from the same subject, and stimulating T cells within the immune cell population by the methods of the invention. This *in vitro* stimulation of T cells, followed by clonal expansion in cell culture of antigen-reactive CD4⁺ and/or CD8⁺ T cells, and administration of the antigen-reactive T cells into the subject, constitute a useful therapeutic and prophylactic strategy. When infused into the subject, antigen-reactive T cells of the invention can specifically target and/or directly kill target cells *in vivo* that bear the same antigen as the antigenic cells, thereby inhibiting cancer development and/or tumor cell proliferation, or preventing or limiting the spread of a pathogen in the recipient antigenic cells, the T cells to be

The antigenic cells, the T cells to be activated, and the recipient of the antigen-reactive T cells may have the same MHC (HLA) haplotype. The invention relates to the use of autologous T cells stimulated *in vitro* with autologously-derived antigen for the treatment of cancer, inhibition of tumor cell proliferation, or prevention of cancer development in the same subject from which the T cells (or more typically, all the immune cells) and antigen were originally derived. In one particular aspect, the immune cells and antigenic cells are isolated from a human subject in need of cellular immunotherapy.

The T cells and the recipient may have the same haplotype while the antigenic cells are allogeneic to the T cells and the recipient, but matched at least one MHC allele, i.e., antigenic cells are used to activate T cells, which T cells are then administered to a recipient from which the T cells were originally obtained, and in which the antigenic cells and the T cells share at least one but not all MHC alleles. In a less typical embodiment of the invention, the antigenic cells, the T cells and the recipient are all allogeneic with respect to each other, but all have at least one common MHC allele shared among the antigenic cells, the T cells and the recipient.

The methods for generating antigen-reactive T lymphocytes may comprise priming live immune cells, pulsing the primed immune cells with BCG and a tissue associated, tissue specific, tumor associated, or tumor specific antigen (with or without LPS), whereas the immune cells comprise APCs, for example, but not limited to dendritic cells, and co-cultured, pulsed cells with primed T cells. The primed immune cells may be enriched for APCs prior to pulsing. The primed immune cells may be separated to generate enriched or purified populations of T cells or APCs. The primed immune cells may be separated to generate enriched or purified populations of CD4⁺ T cells prior to pulsing. Co-culturing of pulsed cells with T cells lead to the stimulation of specific T cells which mature into antigen-reactive CD4⁺ T cells or antigen-reactive CD8⁺ T cells respectively.

Without limitation of the present invention to any particular scientific model or mechanism, the results described herein suggest that BCG with pulsed immune cells (those cells pulsed with a viral antigen, a bacterial antigen, a tissue associated, or tissue specific antigen, a tumor associated or tumor specific antigen, or antigenic fragments thereof as set forth above) comprising APCs are uniquely enabled to induce a specific activation of CD8⁺ T cells *in vitro* directed against virus, bacterial, infected or tumor cells. The results described herein further suggest that a maturation promoting factor can be added to enhance the duration of the immune response. BCG serves to increase DC expression of the surface maturation markers CD83 and CD86, concomitant with exclusion of antigens from endocytosis. Furthermore, lipopolysaccharide (LPS) also down-regulates endocytic activity and promotes DC maturation, potentially increasing the duration of the immune response.

The methods can further comprise restimulation of the antigen reactive T cells *in vitro,* by culturing the cells with feeder cells and irradiated antigenic cells, optionally in the presence of a composition comprising one or more cytokines (*e.g.*, purified IL-2, Concanavalin A-stimulated spleen cell supernatant). *In vitro* restimulation of T cells by addition of APCs and BCG with soluble exogenous antigen, i.e., a viral, a bacterial, a tumor associated antigen or tissue specific antigen or an antigenic fragment of either antigen to the culture can be used to promote expansion of the T cell populations.

The T cells may be stimulated with irradiated spleen cells or APCs purified from peripheral blood as feeder cells in the presence of BCG and viral, bacterial, tissue specific antigen or tumor antigen or an antigenic fragment of either antigen (with or without LPS). In this manner, by restimulation from time-to-time, a stable antigen-specific T cell culture or cell line can be maintained *in vitro* for long periods of time. The T cell culture or cell line thus created can be stored, and if preserved (*e.g*., by formulation with a cryopreservative and freezing) used to resupply antigen-reactive T cells at desired intervals for long term use.

Antigen-reactive CD8⁺ T cells can be generated and used prophylactically to prevent the progression (proliferation of virus, bacteria or tumor cells) or development of a tumor, or to induce remission of cancer. Antigen-reactive CD4⁺ T cells can also be generated and used prophylactically to prevent the progression or development of a tumor (proliferation of tumor cells) or to induce remission of cancer. The T cells can be used therapeutically to treat cancer. Typically, the antigenic cells used to generate the antigen-reactive T cells are syngeneic to the subject to which they are to be administered, *e.g*., are obtained from the subject. However, if antigenic cells that are syngeneic to the subject are not available for use, the methods of the invention provide that such antigenic cells having the same HLA haplotype as the intended recipient of the cells can be prepared *in vitro* using noncancerous cells *(e.g.,* normal cells) collected from the recipient. Lysates or preparations of tumor cells, can be used for restimulation of antigen-reactive T cells of the invention.

Normal cells can be induced to become cancerous or transformed, *e.g.*, by treatment with carcinogens, such as chemicals and/or radiation or infection with a transforming virus, and then used for pulsing directly or used to prepare a lysate for pulsing dendritic cells in combination with BCG or BCG combined with LPS.

Lysates or preparations of such tissue associated or tissue specific; cancerous or transformed cells, and the like, can be used to pulse immune cells or APCs *in vitro.* The lysates or preparations of such cells, can be used for restimulation of the antigen-reactive T cells of the invention.

If the cloned gene of an antigen of interest is available, normal cells from the subject can be transformed or transfected with the gene, such that the antigen of interest is expressed recombinantly in the cells, and then such cells can be used in the priming, pulsing, and/or restimulation reactions. In a less typical aspect, antigenic cells for use can be prepared from cells that are not syngeneic but that have at least one MHC allele in common with the intended recipient.

In an immune response, the process of antigen-induced T cell activation occurs *in vivo* typically in secondary lymphoid tissues, such as the lymph nodes and the spleen. By following the present methods, any antigenic cell of interest can be used to prime T cells *in vitro,* even cancer cells or infected cells that are considered unsafe for use in active immunization. Such primed T cells are then exposed to APCs pulsed with viral, bacterial, tissue specific antigen, tumor antigen, or antigenic fragments of either antigen and BCG. CD8⁺ antigen-reactive T cells can be expanded *in vitro* as a source of cells for immunotherapy. Thus, one advantage of the present methods is that antigen-specific T cells can be expanded *in vitro* to create a source of cells for immunotherapy that can be used for treatment or prevention of disease.

There are many advantages of immunotherapy as provided by the present invention. Tumor bulk is minimal following surgery and immunotherapy is most effective in this situation. The methods of the invention are directed at enhancing the immunocompetence of a cancer patient either before surgery or after surgery, and enhancing cell-mediated tumor-specific immunity against cancer cells, with the objective being inhibition of proliferation of cancer cells, and total eradication of residual cancer cells in the body. In another aspect, antigen-reactive T cells reactive against human cancer cells can be used, alone or in conjunction with surgery, chemotherapy, radiation or other anti-cancer therapies, to eradicate metastases or micrometastases, or to purge bone marrow of cancer cells during bone marrow transplantation. For example, to eradicate or inhibit the growth of metastases or micrometastases, the antigen-reactive T cells provided by the invention, typically CD3⁺CD8⁺ or CD3⁺CD4⁺ T cells are administered *in vivo,* to the subject having or suspected of having a metastases or micrometastases.

As an illustration, to purge bone marrow of cancer cells during bone marrow transplantation, bone marrow from the donor is contacted *in vitro* with the antigen-reactive T cells provided by the invention, so that the antigen reactive T cells lyse any residual cancer cells in the bone marrow, prior to administering the bone marrow to the subject for purposes of hematopoietic reconstitution. The bone marrow transplantation is typically autologous. In one embodiment, the antigen-reactive T cells are CD3⁺CD8⁺ or CD3⁺CD4⁺ T cells. Alternatively, administration of the antigen-reactive T cells involves both CD4⁺ T cells and CD8⁺ T cells.

Moreover, if cancer patients undergo surgery with anesthesia, and subsequent chemotherapy, the resulting immunosuppression experienced by the patient can be lessened by cellular immunotherapy in the preoperative period, thereby reducing the incidence of infectious complications. There is also the possibility that tumor cells are shed into the circulation at surgery, and thus, effective immunotherapy applied at this time can eliminate these cells *in vivo.* The invention thus relates a method of prophylaxis or treatment comprising administering to a cancer patient the antigen-reactive T cells provided by the present invention, reactive against an antigen of the patient's cancer cells, prior to, during, and/or subsequent to surgery and/or chemotherapy undergone by the cancer patient.

A number of antigens or antigenic compositions are useful, according to the present invention, for presentation by the DCs to activate T cells for immunotherapeutics. In one embodiment, a prostate cancer tumor cell lysate recovered from surgical specimens is used as an antigen. For example, a sample of a cancer patient's own tumor, obtained at biopsy or at surgical resection, can be used to provide a cell lysate for antigen. Alternatively, a membrane preparation of tumor cells of a cancer patient, e.g., a prostate cancer patient, or established cell lines can be used as an antigen. Additional antigens useful in the present methods including viral and bacterial antigens, are discussed in detail above.

DCs can be exposed to a desired viral, bacterial, tissue associated or tissue specific antigen, prostate cancer associated antigen, or an antigenic fragment of the antigens by incubating the DCs with the antigen in *in vitro* culture medium. The antigen in aqueous soluble or aqueous suspension form in combination with BCG alone or in combination with BCG and LPS, are added to cell culture medium. As demonstrated herein, the DCs advantageously take up antigen for successful presentation to T cells in the context of MHC-class I.

Antigens can be introduced to the cytosol of the DCs by alternate methods, including, but not limited, to osmotic lysis of pinocytic vesicles and the use of pH sensitive liposomes, and the like. See, generally, (Okada et al., Cell 29:33 (1982); Poste et al., Methods Cell Biol. 14:33 (1976); Reddy et al., J. Immunol. Methods 141:157 (1991)).

### Isolation of Dendritic Cells

Human dendritic cells (DCs) are obtained from any tissue where they reside including non-lymphoid tissues such as the epidermis of the skin (Langerhans cells) and lymphoid tissues such as the spleen, bone marrow, lymph nodes and thymus. DCs can also be isolated as well from the circulatory system including blood (blood DCs) and lymph (veiled cells). Human peripheral blood is an easily accessible ready source of human DCs and is used as a source according to a specific embodiment of the invention. Cord blood is another source of human DCs and in cases where a child is born into a family known to be at high risk for prostate cancer, cord blood can be used as a source of DCs which can be cryopreserved for later use, if needed.

Because DCs occur in low numbers in any tissue in which they reside, including human peripheral blood, DCs must be enriched or isolated for use. Any of a number of procedures entailing repetitive density gradient separation, positive selection, negative selection or a combination thereof can be used to obtain enriched populations or isolated DCs. Examples of such methods for isolating DCs from human peripheral blood include: (O'Doherty et al, J. Exp. Med. 178:1067-1078 (1993); Young and Steinman, J. Exp. Med. 171:1315-1332 (1990); Freudenthal and Steinman, Proc. Natl. Acad. Sci. USA 87:7698-7702 (1990); Macatonia et al., Immunol. 67:285-289 (1989) and Markowicz and Engleman, J. Clin. Invest. 85:955-961 (1990)). A method for isolating DCs from human peripheral blood which avoids exposure of the cells to sheep red blood cells and/or fetal calf serum is described in PCT Publication W094/02156. An example of a method for isolating DCs from lymphoid tissue is described in (Macatonia et al., J. Exp. Med. 169:1255-1264 (1989)).

Once the DCs are obtained, they are cultured in appropriate culture medium to expand the cell population and/or to maintain the DCs in a state for optimal antigen uptake, processing and presentation.

According to one embodiment, which also provides maintenance of the proper state of "maturity" of DCs in *in vitro* culture, is to culture the DCs the presence of both granulocyte/macrophage colony stimulating factor (GM-CSF) and interleukin 4 (IL-4). In one example, a combination of GM-CSF and IL-4 at a concentration of about 500 units/ml of each. A recent study reveals optimal antigen presentation by "immature" vs. mature DCs (Koch et al., J. Immunol. 155:93-100 (1995)). Immature DCs may be used according to certain embodiments of the present invention. Recent experiments have shown that mature pulsed DCs retain the ability to stimulate a T cell response up to ten times longer than immature pulsed DCs.

As illustrated in the examples, *infra*, human DCs were isolated from peripheral blood of prostate cancer patients, and after about 5 days in *in vitro* culture, DCs competent and able to activate prostate cancer specific T cells were recovered. According to one embodiment of the invention, DCs are obtained from a cancer patient to be treated. The DCs are pulsed with one of the various antigens provided herein in the presence of BCG with or without LPS, and then used to activate autologous T cells of the patient, either *in vitro* or *in vivo,* for cancer immunotherapy and/or tumor growth inhibition.

According to an alternative embodiment, DCs are obtained from a healthy individual known not to be suffering from cancer. The relevant HLA antigens (both MHC class I and II, e.g., HLA-A, B, C and DR) on the individual's peripheral blood mononuclear cells (PBMCs) are identified and DCs which provide an HLA match with the cancer patient are isolated and expanded as described above. For example, in certain instances, late stage prostate cancer patients who have been treated with radiation and/or chemotherapy agents often are not able to provide sufficient or efficient DCs. Thus, DCs from healthy HLA-matched individuals, such as siblings, can be obtained and expanded using any of the methods described above and incubated *in vitro* with a relevant antigen in the presence of BCG to form activated natural DCs which in turn can be used to elicit activated T cells for immunotherapy and/or to inhibit tumor growth in the HLA-matched prostate cancer patient

According to another embodiment of the invention, "extended life span dendritic cells" are used. Human cells have a finite life span *in vitro* usually limited to approximately 50-70 population doublings before undergoing apoptosis. As used herein, the term "extended life span dendritic cells" is intended to mean DCs that have been genetically modified so that they can be expanded in *in vitro* cell culture medium for an extended period of time, including but not limited to at least about 100 additional population doublings. Extended life span DCs are obtained, for example, by EBV-transformation of DCs obtained from peripheral blood of prostate cancer patients, or by insertion into DCs, using techniques known to those skilled in the art, of a specific cell cycle regulatory gene including but not limited to a gene which encodes cyclin A, B, D or E, or retinoblastoma protein.

As illustrated in the examples presented in U.S. Patent No. 5,788,963 extended life span DCs have been obtained by EBV transformation of a population of isolated DCs. Such extended life span DCs are useful according in the methods of the present invention.

According to another embodiment of the invention, DCs can be preserved, *e.g.,* by cryopreservation either before exposure or following exposure to a relevant antigen. Cryopreservation agents which can be used include but are not limited to dimethyl sulfoxide (DMSO) (Lovelock and Bishop, Nature 183:1394-1395 (1959); Ashwood-Smith, Nature 190:1204-1205 (1961)), glycerol, polyvinylpyrrolidone (Rinfret, N.Y. Acad. Sci. 85:576 (1960)), polyethylene glycol (Sloviter and Ravdin, Nature 196:548 (1962)), albumin, dextran, sucrose, ethylene glycol, i-erythritol, D-ribitol, D-mannitol (Rowe et al., Fed. Proc. 21:157-(1962)), D-sorbitol, i-inositol, D-lactose, choline chloride (Bender et al., J. Appl. Physiol. 15:520 (1960), amino acids (Phan and Bender, Exp. Cell Res. 20:651 (1960)), methanol, acetamide, glycerol monoacetate (Lovelock, Biochem. J. 56:265 (1954)), and inorganic salts (Phan and Bender, Proc. Soc. Exp. Biol. Med. 104:388 (1960); Phan and Bender, in Radiobiology, Proceedings of the Third Australian Conference on Radiobiology, Ilbery, P.L.T., ed., Butterworth, London, p. 59 (1961)).

A controlled slow cooling rate is critical. Different cryoprotective agents (Rapatz et al., Cryobiology 5:18-25 (1968)) and different cell types have different optimal cooling rates (see, *e.g*., Rowe and Rinfret, Blood 20:636 (1962); Rowe, Cryobiology 3:12-18 (1966); Lewis et al., Transfusion 7:17-32 (1967); and Mazur, Science 168:939-949 (1970)), for effects of cooling velocity on survival of marrow-stem cells and on their transplantation potential). The heat of fusion phase where water turns to ice should be minimal. The cooling procedure can be carried out by use of, *e.g*., a programmable freezing device or a methanol bath procedure. Programmable freezing apparatuses allow determination of optimal cooling rates and facilitate standard reproducible cooling. Programmable controlled-rate freezers such as Cryomed or Planar permit tuning of the freezing regimen to the desired cooling rate curve.

After thorough freezing, cells can be rapidly transferred to a long-term cryogenic storage vessel. In a particular embodiment, samples can be cryogenically stored in liquid nitrogen (-196°C) or its vapor (-165°C). Such storage is greatly facilitated by the availability of highly efficient liquid nitrogen refrigerators.

Considerations and procedures for the manipulation, cryopreservation, and long term storage of hematopoietic stem cells, particularly from bone marrow or peripheral blood, is largely applicable to the DCs prepared by the methods of the invention. Such a discussion can be found, for example, in the following references, incorporated by reference herein: (Gorin, Clinics in Haematology 15:19-48 (1986); Bone-Marrow Conservation, Culture and Transplantation, Proceedings of a Panel, Moscow, July 22-26, 1968, International Atomic Energy Agency, Vienna, pp. 107-186)). Other methods of cryopreservation of viable cells, or modifications thereof, are available and envisioned for use (e.g., cold metal-mirror techniques; Livesey and Linner, Nature 327:255 (1987); Linner et al., J. Histochem. Cytochem. 34:1123-1135 (1986); see also U.S. Patent No. 4,199,022 to Senken et al., U.S. Patent No. 3,753,357 to Schwartz, U.S. Patent No. 4,559,298 to Fahy, and U.S. Patent No. 5,364,756 to Livesey, et al.

Frozen cells are typically thawed quickly (*e.g*., in a water bath maintained at 37-41°C) and chilled immediately upon thawing. It may be desirable to treat the cells in order to prevent cellular clumping upon thawing. To prevent clumping, various procedures can be used, including but not limited to, the addition before and/or after freezing of DNase (Spitzer et al., Cancer 45:3075-3085 (1980)), low molecular weight dextran and citrate, hydroxyethyl starch (Stiff et al., Cryobiology 20:17-24 (1983)), and the like. The cryoprotective agent, if toxic in humans, should be removed prior to administration of the thawed DCs to an individual. One way in which to remove the cryoprotective agent is by dilution to an insignificant concentration. Once frozen DCs have been thawed and recovered, they are used to activate T cells as described above with respect to non-frozen DCs.

### Applications or Methods of Use

Solely for the ease of presentation, the following discussion is illustrative of particular uses of the methods of the invention as would be clearly understood by those with skill in the relevant art. The examples provided herein are not meant to limit the scope of the present invention which can be used for essentially any antigen, including virus and bacteria for all cancer types.

As discussed *supra*, according to one embodiment of the invention, isolated human DCs, exposed to a soluble exogenous prostate specific antigen and BCG by any of the methods disclosed herein can be used to activate T cells *in vitro* against prostate cancer. In particular, the T cell response is a MHC class I-directed response providing a population of activated T cells comprising greater than 25% CD8⁺T cells. The DCs can be used immediately after exposure to antigen to stimulate T cells. Alternatively, the DCs can be maintained in the presence of a combination of GM-CSF and IL-4 prior to simultaneous exposure to antigen and T cells.

T cells or a subset of T cells can be obtained from various lymphoid tissues for use as responder cells. Such tissues include but are not limited to spleen, lymph nodes, and peripheral and cord blood. The cells can be co-cultured with DCs exposed to antigen as a mixed T cell population or as a purified T cell subset For example, it may be desired to culture purified CD8⁺ T cells with antigen exposed DCs to elicit prostate specific cytotoxic T lymphocytes (CTLs). In addition, early elimination of CD4⁺ T cells during *in vitro* cell culture can prevent the overgrowth of CD4⁺ cells in a mixed culture of both CD8⁺ and CD4⁺ T cells. T cell purification can be achieved by positive, and/or negative selection, including but not limited to, the use of antibodies directed to CD2, CD3, CD4, CD8, and the like.

According to one embodiment, the T cells are obtained from the same prostate cancer patient from which the DCs were obtained. After stimulation or activation *in vitro,* the autologous T cells are administered to the patient to provoke and/or augment an existing immune response which slows or inhibits prostate cancer tumor growth. For example, T cells can be administered, by intravenous infusion, at doses of about 10⁸-10⁹ cells/m² of body surface area (see, Ridell et al., Science 257:238-241 (1992)). Infusion can be repeated at desired intervals, for example, monthly. Recipients are monitored during and after T cell infusions for any evidence of adverse effects.

According to another embodiment, the T cells are obtained from a prostate cancer patient and the DCs which are used to stimulate the T cells are obtained from an HLA-matched healthy donor. According to yet another embodiment, both the T cells and the DCs are obtained from an HLA-matched healthy donor, *e.g*., a sibling of the prostate cancer patient. This embodiment can be advantageous, for example, when the patient is a late stage prostate cancer patient who has been treated with radiation and/or chemotherapy agents and may not be able to provide sufficient or efficient DCs. The T cells after stimulation, are administered as described above.

In a specific example of the methods of the present invention, PSMA loaded into dendritic cells (DCs) using various methodologies produced antigen-presenting cells that can stimulate autologous and allogeneic T cells in an antigen-specific manner. These methodologies include: 1) overnight treatment of about day 6 DCs with PSMA protein and bacillus Calmette-Guérin mycobacteria (BCG) with or without lipopolysaccharide (LPS), and 2) osmotic loading of about day 7 DCs using hypertonic medium. BCG stimulated DCs demonstrate elevated CD83 and CD86 expression while LPS further enhances DC maturation. Osmotic loading was accomplished using hypertonic medium to increase phagocytosis and macropinocytosis.

Following two weekly stimulations *in vitro* with PSMA-loaded DCs (prepared using BCG ± LPS or osmotic loading) and one or more weekly restimulations with PBMC exogenously pulsed with PSMA, specific reactivity to the immunogen was demonstrated (Fig. 1-6).

According to another embodiment of the invention, DCs isolated from a prostate cancer patient are cultured *in vitro,* then exposed to a prostate tissue specific antigen, a prostate cancer antigen, or an antigenic fragment of either antigen in a manner sufficient to obtain MHC-class I antigen presentation and which increases the relative number of CD8⁺ CTLs. After either expansion or cryopreservation, DCs are administered back to the patient to stimulate an immune response, including T cell activation, against the patient's cancer cells *in vivo.* Using this approach with the patient's own dendritic cells provides the following advantages: (1) no foreign DNA is utilized; (2) infection of cells for purposes of cDNA expression using various viral vectors are eliminated; (3) antigen is presented to dendritic cells in the form of soluble protein which will be taken into the dendritic cells and processed for MHC/peptide presentation of the cell surface; (4) dendritic cells express B7 on their surface alleviating the necessity to transfect this cDNA into dendritic cells; (5) the use of endogenous B7 (either B7.1 and/or B7.2) on the dendritic cell surface eliminates the need to provide T cells with IL-2 or other cytokines either in the form of the cytokine itself or transfection of the cDNA into specific cells; (6) all procedures are carried out using the patient's own cells.

DCs obtained as described above, are exposed *in vitro* to a prostate specific antigen, a prostate cancer antigen or an antigenic fragment of either antigen (*e.g*., PSMA at about 0.1 µg to about 1000 µg) in combination with BCG (approximately 2 x 10⁵ to about 1 x 10⁶ Units/ml final concentration) or BCG in combination with LPS (40 Units/ml), washed and administered to a patient to elicit an immune response or to augment an existing immune response. As such, the DCs constitute an anti-prostate cancer vaccine and/or immunotherapeutic agent. DCs presenting a prostate specific antigen are administered to a patient, via intravenous infusion, at a dose of about 10⁶-10⁸ cells. The immune response of the patient can be monitored. Infusion can be repeated at desired intervals based upon the patient's measured immune response.

The following examples demonstrate that human dendritic cells, obtained from prostate cancer patients, pulsed with antigen, for example in the form of autologous tumor lysate or peptide in the presence of a combination of BCG and LPS, or with BCG alone, stimulate antigen-specific cytotoxic T cell and humoral immune responses to human prostate cancer antigens. These examples are presented for purposes of illustration only and are not intended to limit the scope of the invention in any way.

### EXAMPLE 1

The following example describes the isolation and culturing of human dendritic cells. Isolated dendritic cells were contacted with tumor cell lysate, and partially purified tumor cell lysate in combination with BCG to demonstrate the stimulation of antigen-specific cytotoxic T cell response.

### Culture of Patient Dendritic Cells

Cultures of human DCs were established as described previously herein and in U.S. Patent No: 5,788,963. Briefly, peripheral blood mononuclear cells (PBMC) were obtained from leukocyte-enriched "buffy coats" by standard centrifugation on Ficoll-Paque (Pharmacia, Uppsala, Sweden). Plastic-adherent PBMCs (about 1 hour at 37°C) were cultured for 6 to 7 days in AIM-V either supplemented or not with 2% autologous serum, 50 U/ml penicillin, 50 µg/ml streptomycin, 2 mM L-glutamine, 10 mM HEPES, 0.1 mM non-essential amino acids, and 1 mM pyruvate (referred to as "culture medium"; all from Boehringer Ingelheim, Biowhittaker, Verviers, Belgium) in the presence of 1000 U/ml or 500 U/ml of each granulocyte macrophage-colony stimulating factor (GM-CSF) (Leucomax^{™} 1.11 x 10⁷ U/mg from Novartis, Basel, Switzerland) and 500 U/ml Interleukin-4 (IL-4) (Schering-Plough Research Institute, Kenilworth, NJ).

### Phenotyping of Patient DCs

To determine surface antigen (Ag) expression, cells (10⁵ DCs in 50 µl) were labeled with primary monoclonal antibody (mAb) in complete medium followed by FITC-conjugated F(ab')₂ fragments of goat anti-mouse Ig (Dako, Glostrup, Denmark). The following monoclonal antibodies can be used although numerous others having the same specificity are well known: G46-2.6 (IgG₁, anti-HLA-ABC), L243 (IgG₂ₐ, anti-HLA-DR), HB-15a (IgG_{2b}, anti-CD83), BU63 (IgG₁, anti-CD86). Washes were in HBSS containing 0.2% albumin. After the last wash, the cells were stored in HBSS containing 0.2% albumin and 2% formaldehyde. The samples were analyzed on a FACScan^{®} (Becton-Dickinson, San Jose, CA). Data was analyzed and presented using CellQuest^{®} software from Becton-Dickinson.

### Preparation of Tumor Cell Lysates

BCG and LPS were used to stimulate MHC-class I loading of DCs as follows: 1-100 µg of LNCaP-derived PSMA or partially purified recombinant PSMA (rPSMA) was added to the culture medium of day 6 DCs by pipetting with a sterile microtip. At the same time, BCG (0.2 - 1.6 x 10⁶ U/ml final concentration; Tice-BCG, Organon Teknika, Durham, NC) and, in a replicate flask, LPS (40 U/ml final concentration) was added to the culture medium using the same method. The culture medium was then mixed gently before being returned to a CO₂ incubator.

Osmotic loading was performed as follows: day 7 DCs were harvested by vigorous pipetting with phosphate buffered saline (PBS), then incubated with PBS preceding mechanical dislodging. DCs were centrifuged at low speed, and all supernatant was removed using aspiration. The cell pellets were resuspended in the desired amount of PSMA and PBS to a final volume of 100 µl. Subsequently, 100 µl hypertonic medium (1 M sucrose and 20% glycerol) was added, and the cell suspension mixed with a pipet microtip. The cells were then placed in a 37°C water bath for 10 minutes, in a 15 ml centrifuge tube. Following the incubation, isotonic conditions were restored by filling the tube with DMEM or AIM-V, then returning the tube to the 37°C water bath for an additional 3 minutes. Cells were then centrifuged and resuspended in culture medium at the desired concentration.

Following two weekly stimulations *in vitro* with PSMA loaded DCs and one or more weekly restimulations with PBMC exogenously pulsed with PSMA, specific reactivity to the immunogen was demonstrated (Fig. 1-5). By day 18 of culture, effector cells from Patient 92 specifically recognized autologous DCs presenting PSMA but not the irrelevant protein ovalbumin (OVA) or untreated DCs (Fig. 1A). Patient 92 effector cells also specifically recognized allogeneic DCs from Patient 105 and I.T. (Fig. 1B and Fig. 1C). Effector cells from both Patient 105 and I.T. secreted cytokine in a specific manner following cocultivation with both autologous and allogeneic DCs presenting PSMA.

The observed PSMA-restricted activity contains a significant CD8⁺ T cell component (Fig. 2A - Fig. 2C). As illustrated, when the effector cells from patient 105 were stimulated using DCs in which PSMA had been loaded in the presence of BCG, cytokine secretion was significantly CD8-mediated, regardless of whether LPS was included during stimulations (Fig. 2A and Fig. 2B). When the effector cells were stimulated using DCs expressing PSMA following osmotic loading, there was not a statistically significant difference in the relative contribution of CD8⁺ and CD4⁺ T cells to the observed reactivity (Fig. 2C). Similar findings were obtained using identically stimulated effectors from patients 92 and I.T. (data not shown). These results demonstrate that use of BCG, with or without LPS, as presently taught, yields an immune response biased towards cytotoxic T cells (i.e., over 50% of T cells were CD8⁺).

Following additional restimulations with PSMA-loaded PBMC (using osmotic loading), specific effectors from patient 105 were evaluated to determine whether these effectors had the ability to secrete cytokines in a dose-dependent manner (Fig. 3). For the initial stimulations at days 0 and 10, if BCG or BCG + LPS was present during PSMA loading into DCs the amount of cytokine secreted by the effectors was directly associated with the amount of PSMA osmotically loaded into autologous DC targets (Fig. 3A and 3B). The greatest amount of IFNy secretion was observed when the highest concentration of PSMA tested was loaded into DCs, without a maximum being reached. All stimulations *in vitro* were performed using DCs or PBMC loaded with 10 µg of PSMA. This amount of protein was sufficient for strong T cell stimulation and activation. When the experiment was repeated with the maximum concentration of PSMA increased to 30 µg, a plateau was achieved (Fig. 3C).

A moderate level of PSMA specific cytolysis can be detected (Fig. 4A and 4B). At 32 days of culture, effector cells from Patient I.T. displayed 37% lysis of autologous DCs presenting PSMA at an effector:target ratio of 40:1, as compared with 23% against autologous DCs presenting OVA or 19% against untreated targets (Fig. 4A). At 39 days of culture, effector cells from Patient 92 displayed 23% lysis of lysis of autologous DCs presenting PSMA at an effector:target ratio of 36:1, as compared with 14% against autologous DCs presenting OVA or 10% against untreated targets (Fig. 4B).

Specificity of PSMA-derived peptides recognized by T cells stimulated *in vitro* with DCs osmotically loaded with intact, soluble protein was also determined. Using effector cells from Patient 92 that were PSMA-specific following stimulation *in vitro,* cytokine secretion was measured after co-culturing with the antigen-presenting cell line T2 exogenously pulsed with one of the HLA-A2⁺ peptides derived from PSMA:PSM-P1 or influenza M1 protein (Fig. 6A and Fig. 6B).

In an additional experiment it was demonstrated that dendritic cells osmotically or directly loaded with influenza M1 protein or peptide, comprising an antigen fragment and matured in the presence of either BCG alone or in combination with interferon gamma are capable of stimulating a T cell mediated activity as measured by the production of interferon gamma by the V_{β17} T cell subset.

### EXAMPLE 2

The present example examines whether dendritic cells retain their function after cryopreservation. This characteristic is particularly important because immunotherapy approaches involve multiple treatments and it is preferable that all the DCs for each patient be prepared and loaded with antigen during a single preparation, then aliquoted and cryopreserved for subsequent infusion. It was possible that the freezing and thawing of the DCs may limit their effectiveness as CD8⁺ T cell activators.

Dendritic cells were isolated from PBMC of a prostate cancer patient and cultured, as described above, for 7 days in the presence of 500 U/ml GM-CSF and 500 U/ml IL-4. On day 7, the isolated DC's were harvested and cryopreserved using 90% fetal calf serum and 10% dimethylsulfoxide. The cryopreserved DC's were subsequently stored frozen for a period of time, thawed in a 37°C water bath and transferred to a 15 ml polypropylene tube and centrifuged at 1200 rpm for 5 min. The thawed DC's were then resuspended in medium containing 10% heat-inactivated human serum and counted and used as described below.

Day 7 DCs from Patient 105 were osmotically loaded with PSMA or OVA (or left untreated). The treated DCs were then frozen in a standard freeze medium of 90% human serum and 10% DMSO as described, *supra.* On the same day, a second set of DC cultures was established. One week later, fresh day 7 DCs were harvested and osmotically loaded with PSMA or OVA (or left untreated). In addition, DCs that had been cryopreserved the week before were thawed by standard techniques and used immediately in an ELISA. PSMA-restricted effector cells from Patient 105 displayed strong reactivity to both fresh and cryopreserved targets (Fig. 5). As taught in this specific example, cryopreservation does not diminish the effectiveness of DCs to function as antigen presenting cells.

Inciting a potent anti-tumor response using immunotherapy has been limited in efficacy partly due to difficulty in stimulating a cytotoxic T cell response. The present invention describes methods and compositions to overcome this limitation. The invention entails exposing dendritic cells to soluble tissue specific antigen in the presence ofBacille Calmette Guerin (BCG), such that the BCG helps direct the antigen into the MHC-class I processing pathway inducing a predominantly cytotoxic T cell response. Recent work by Cella and colleagues (Cella et al., Nature 388:782-787 (1997)), has also demonstrated the importance of the maturation process for antigen presentation. In the absence of inflammatory stimuli, the half life of an antigen presented on MHC-class II molecules on DCs was 10 hours. In contrast, pulsing the DCs with antigen in the presence of inflammatory factors increased the half life of the antigen to 100 hours. The longer half life allows the DCs to home to secondary lymphoid organs and to activate antigen-specific T-lymphocytes.

### EXAMPLE 3

In this example dendritic cells isolated from a cancer patient were isolated and treated with various concentrations of BCG. After several days of culture, the DCs were tested for 1) the capacity to uptake particles by pinocytosis, and 2) the surface expression of certain dendritic cell maturation markers, including HLA-DR, CD86, CD40, CD83, CD80 and HLA-class I.

Dendritic cells were isolated from patient 57 as described above. The isolated cells (1-5 x 10⁶) were cultured for about 6 days in eight T-75 flasks. BCG (1 x 10⁶ units/ml) was added to duplicate flasks to achieve a dilution of 1:250, 1:2,500, or 1:25,000. No BCG was added to the two remaining culture flasks. A first set of culture flasks comprising DCs with no BCG, or with 1:250; 1:2,500: or 1:25,000 BCG dilution was harvested after a 48 or 72 hour incubation. The duplicate set of culture flasks was harvested after a total of 72 hours in culture. Each DC culture was analyzed for: (1) the capacity to uptake FITC/Dextran by pinocytosis, and (2) the level of surface expression of particular DC maturation markers, including HLA-DR, CD86, CD40, CD83, CD80, and HLA-class I.

The capacity to uptake labeled dextran particles by pinocytosis was tested as follows: Each DC culture flask contents was aliquoted into duplicate tubes and incubated in AIM-V medium on ice for 30 minutes. Following incubation on ice, FITC/Dextran was added to each tube to achieve a final concentration of approximately 1 mg/ml. One set of tubes was incubated at 37°C, in 5% CO₂, for 1 hour, while the duplicate set of tubes was incubated on ice (0°C) for approximately 1 hour. DCs were washed 3 times in phosphate buffered saline (PBS) prior to analysis by flow cytometry. The relative amount of pinocytosis by DCs was compared after subtracting background uptake at 0°C (Table 2).

For analysis of DC maturation markers each DC culture was incubated with the following monoclonal antibody pairs: FITC-anti HLA-DR/PE-anti-CD86; FITC-anti CD40/PE - anti-CD83; FITC-anti-CD80/PE-anti-HLA-class I; or FITC-/PE-isotype antibody controls using standard methods. Surface expression of each DC markers were analyzed by flow cytometry (Table 3).

**Table 2**

| **FITC - Dextran Uptake (Mean Fluorescence 37°C - Mean Fluorescence 0°C)** | | | | | |
|---|---|---|---|---|---|
| **Exp. No.** | **BCG concentration** | **Duration (h)** | **Mean** | **GEO Mean** | **Median** |
| 1 | 0 | 48 | 51 | 36.3 | 39.15 |
| 2 | 1:250 | 48 | 28.09 | 20.76 | 22.03 |
| 3 | 1:2500 | 48 | 37.02 | 27.11 | 29.28 |
| 4 | 1:25000 | 48 | 40.44 | 34.12 | 38.04 |
| 5 | 0 | 72 | 63.8 | 43.2 | 47.3 |
| 6 | 1:250 | 72 | 34.76 | 22.85 | 22.6 |
| 7 | 1:2500 | 72 | 49.79 | 36.24 | 39.6 |
| 8 | 1:25000 | 72 | 55.62 | 38.27 | 41.41 |

**Table 3**

| **Mean FL difference** | | | | | | | | |
|---|---|---|---|---|---|---|---|---|
| **Exp. No.** | **BCG concentration** | **Duration (h)** | **HLA-DR FITC** | **CD86 PE** | **CD40 FITC** | **CD83 PE** | **CD80 FITC** | **HLA-Class** I **PE** |
| 1 | 0 | 48 | 250 | 613.07 | 19.64 | 15.14 | -1.1 | 140.28 |
| 2 | 1:250 | 48 | 276.54 | 957.38 | 24.78 | 62.58 | -1.27 | 173.69 |
| 3 | 1:2500 | 48 | 267.94 | 734.1 | 21.9 | 21.4 | -0.94 | 135.45 |
| 4 | 1:25000 | 48 | 277.1 | 632 | 18.2 | 17.59 | -1.62 | 148.97 |
| | | | | | | | | |
| 5 | 0 | 72 | 337.13 | 705.75 | 20.89 | 22.42 | -1.26 | 193.06 |
| 6 | 1:250 | 72 | 331.71 | 1106.12 | 33.39 | 127.65 | -2.11 | 373.06 |
| 7 | 1:2500 | 72 | 336.87 | 694.74 | 24.2 | 35.1 | -1.41 | 177.6 |
| 8 | 1:25000 | 72 | 298.96 | 646.95 | 19.81 | 24.25 | -1.15 | 170.26 |

At a concentration of 1:250 DCs showed a significant increase in CD86, CD40, CD83 and HLA-class I in both the 48 and 72 hour cultures. The effect of BCG was more pronounced at 72 hours. For example, HLA-class I increased by 24 % after 48 hours in 1:250 BCG, but increased 93 % after 72 hours of 1:250 BCG. Likewise, CD83 increased by 40 fold at 48 hours in 1:250 BCG and 5.7 fold after 72 hours in 1:250 BCG.

## Claims

1. A method for promoting Major Histocompatibility Complex-(MHC-) class I processing of an antigen by human dendritic cells (DCs), wherein said DCs are exposed, *in vitro,* to an antigen associated with a tumor cell in the presence of bacillus Calmette Guerin (BCG) or BCG with lipopolysaccharide (LPS) as a factor or agent that promotes MHC-class I processing of the antigen.

2. The method according to claim 1, in which the antigen is a lysate of tumor cells isolated from a patient, a purified tumor associated antigen, a purified tissue associated antigen, or an antigenic fragment thereof.

3. The method according to claim 2, wherein the antigen is a prostate tumor associated antigen.

4. The method according to claim 3, wherein the prostate tumor associated antigen is purified prostate specific membrane antigen (PSMA).

5. The method according to any of claims 1 to 4, in which the human DCs were obtained from the skin, spleen, bone marrow, thymus, lymph node, chord blood, or peripheral blood.

6. The method according to any of claims 1 to 5, in which the dendritic cells are extended life span dendritic cells.

7. A method for producing activated T cells *in vitro* by
a) providing isolated DC's to which has been added
- BCG at a concentration of 1x10⁶ units/ml to achieve a dilution selected from the group consisting of 1:250, 1:2,500, and 1:25,000, and
- an antigen associated with a tumor cell, and
b) contacting the T cells in vitro with said DC's.

## Patentansprüche

1. Ein Verfahren zur Förderung der Haupthistokompatibilitäts-komplex-(MHC-)Klasse I-Prozessierung eines Antigens durch humane dendritische Zellen (DCs), wobei die DCs *in vitro* einem Antigen, welches mit einer Tumorzelle in Zusammenhang steht, und in Anwesenheit des Calmette-Guerin-Bazillus (BCG) oder BCG mit Lipopolysaccharid (LPS) als Faktor oder Mittel welches die MHC-Klasse I Antigenprozessierung fördert, ausgesetzt sind.

2. Das Verfahren nach Anspruch 1, wobei das Antigen ein von einem Patienten isoliertes Tumorzellenlysat, ein aufgereinigtes tumorassoziiertes Antigen, ein aufgereinigtes gewebeassoziiertes Antigen oder ein Antigenfragment davon ist.

3. Das Verfahren nach Anspruch 2, wobei das Antigen ein prostatatumorassoziiertes Antigen ist.

4. Das Verfahren nach Anspruch 3, wobei das Prostatatumorassoziierte Antigen aufgereinigtes prostataspezifisches Membranantigen (PSMA) ist.

5. Das Verfahren nach einem der Anspruch 1 bis 4, wobei die humanen DCs aus der Haut, Milz, Knochenmark, Thymus, Lymphknoten, Nabelschnurblut oder peripheren Blut erhalten wurden.

6. Das Verfahren nach einem der Anspruch 1 bis 5, wobei die dendritischen Zellen solche mit verlängerter Lebensdauer sind.

7. Ein Verfahren zur Herstellung aktivierter T-Zellen *in vitro* durch
a) die Bereitstellung isolierter DCs, zu denen
- BCG in einer Konzentration von 1 x 10⁶ Einheiten/ml hinzugefügt wurde um eine Verdünnung ausgewählt aus der Gruppe bestehend aus 1:250, 1:2500 und 1:25000 zu erreichen, und
- ein mit einer Tumorzelle in Zusammenhang stehendes Antigen hinzugefügt wurde, und
b) das Kontaktieren der T-Zellen *in vitro* mit den DCs.

## Revendications

1. Méthode pour favoriser le traitement par le complexe majeur d'histocompatibilité (CMH) de classe I d'un antigène par les cellules dendritiques (CD) humaines, dans laquelle lesdites CD sont exposées, *in vitro,* à un antigène associé à une cellule tumorale en présence du bacille de Calmette-Guérin (BCG) ou du BCG avec un lipopolysaccharide (LPS) comme facteur ou agent qui favorise le traitement de l'antigène par le CMH de classe I.

2. Méthode selon la revendication 1, dans laquelle l'antigène est un lysat de cellules tumorales isolées à partir d'un patient, un antigène purifié associé à une tumeur, un antigène purifié associé à un tissu, ou un fragment antigénique de ceux-ci.

3. Méthode selon la revendication 2, dans laquelle l'antigène est un antigène associé à une tumeur de la prostate.

4. Méthode selon la revendication 3, dans laquelle l'antigène associé à une tumeur de la prostate est l'antigène membranaire spécifique de la prostate (PSMA) purifié.

5. Méthode selon l'une quelconque des revendications 1 à 4, dans laquelle les CD humaines ont été obtenues à partir de la peau, de la rate, de la moelle osseuse, du thymus, des ganglions lymphatiques, du sang de cordon ombilical, ou du sang périphérique.

6. Méthode selon l'une quelconque des revendications 1 à 5, dans laquelle les cellules dendritiques sont des cellules dendritiques à durée de vie prolongée.

7. Méthode *in vitro* pour produire des lymphocytes T activés par
(a) la fourniture de CD isolées auxquelles a été ajouté
- du BCG à une concentration de 1 x 10⁶ unités/ml pour obtenir une dilution choisie dans le groupe constitué de 1/250, 1/2 500, et 1/25 000, et
- un antigène associé à une cellule tumorale, et
(b) la mise en contact des lymphocytes T *in vitro* avec lesdites CD.


### SEQUENCE LISTING

<110> Salgaller, Michael L. Boynton, Alton L. NORTHWEST BIOTHERAPEUTICS, INC.
<120> METHOD TO INCREASE CLASS I PRESENTATION OF EXOGENOUS ANTIGENS BY HUMAN DENDRITIC CELLS
<130> 20093-8-1PC
<140> PCT/US01/
   <141> 2001-05-11
<150> 60/203,758
   <151> 2000-05-12
<160> 37
<170> PatentIn Ver. 2.1
<210> 1
   <211> 9
   <212> PRT
   <213> Homo sapiens
<400> 1
   Leu Leu His Glu Thr Asp Ser Ala Val
   1 5
<210> 2
   <211> 9
   <212> PRT
   <213> Homo sapiens
<400> 2
   Ala Leu Phe Asp Ile Glu Ser Lys Val
   1 5
<210> 3
   <211> 9
   <212> PRT
   <213> Homo sapiens
<400> 3
   Trp Leu Cys Ala Gly Ala Leu Val Leu
   1 5
<210> 4
   <211> 9
   <212> PRT
   <213> Homo sapiens
<400> 4
   Val Leu Ala Gly Gly Phe Phe Leu Leu
   1 5
<210> 5
   <211> 9
   <212> PRT
   <213> Homo sapiens
<400> 5
   Glu Leu Ala His Tyr Asp Val Leu Leu
   1 5
<210> 6
   <211> 9
   <212> PRT
   <213> Homo sapiens
<400> 6
   Asn Leu Asn Gly Ala Gly Asp Pro Leu
   1 5
<210> 7
   <211> 9
   <212> PRT
   <213> Homo sapiens
<400> 7
   Thr Leu Arg Val Asp Cys Thr Pro Leu
   1 5
<210> 8
   <211> 9
   <212> PRT
   <213> Homo sapiens
<400> 8
   Val Leu Arg Met Met Asn Asp Gln Leu
   1 5
<210> 9
   <211> 9
   <212> PRT
   <213> Homo sapiens
<400> 9
   Pro Met Phe Lys Tyr His Leu Thr Val
   1 5
<210> 10
   <211> 9
   <212> PRT
   <213> Homo sapiens
<400> 10
   Asn Met Lys Ala Phe Leu Asp Glu Leu
   1 5
<210> 11
   <211> 9
   <212> PRT
   <213> Homo sapiens
<400> 11
   Leu Met Tyr Ser Leu Val His Asn Leu
   1 5
<210> 12
   <211> 9
   <212> PRT
   <213> Homo sapiens
<400> 12
   Met Met Asn Asp Gln Leu Met Phe Leu
   1 5
<210> 13
   <211> 9
   <212> PRT
   <213> Homo sapiens
<400> 13
   Glu Gly Asp Leu Val Tyr Val Asn Tyr
   1 5
<210> 14
   <211> 9
   <212> PRT
   <213> Homo sapiens
<400> 14
   Ala Gly Asp Pro Leu Thr Pro Gly Tyr
   1 5
<210> 15
   <211> 9
   <212> PRT
   <213> Homo sapiens
<400> 15
   Arg Val Asp Cys Thr Pro Leu Met Tyr
   1 5
<210> 16
   <211> 9
   <212> PRT
   <213> Homo sapiens
<400> 16
   Leu Phe Glu Pro Pro Pro Pro Gly Tyr
   1 5
<210> 17
   <211> 9
   <212> PRT
   <213> Homo sapiens
<400> 17
   Thr Tyr Glu Leu Val Glu Lys Phe Tyr
   1 5
<210> 18
   <211> 9
   <212> PRT
   <213> Homo sapiens
<400> 18
   Ala Gly Glu Ser Phe Pro Gly Ile Tyr
   1 5
<210> 19
   <211> 9
   <212> PRT
   <213> Homo sapiens
<400> 19
   Trp Gly Glu Val Lys Arg Gln Ile Tyr
   1 5
<210> 20
   <211> 11
   <212> PRT
   <213> Homo sapiens
<400> 20
   Ile Val Arg Ser Phe Gly Thr Leu Lys Lys Glu
   1 5 10
<210> 21
   <211> 11
   <212> PRT
   <213> Homo sapiens
<400> 21
   Asp Glu Leu Lys Ala Glu Asn Ile Lys Lys Phe
    1               5                  10
<210> 22
   <211> 11
   <212> PRT
   <213> Homo sapiens
<400> 22
   Lys Ser Leu Tyr Glu Ser Trp Thr Lys Lys Ser
    1               5                  10
<210> 23
   <211> 9
   <212> PRT
   <213> Homo sapiens
<400> 23
   Ala Tyr Ile Asn Ala Asp Ser Ser Ile
    1               5
<210> 24
   <211> 9
   <212> PRT
   <213> Homo sapiens
<400> 24
   Lys Tyr Ala Asp Lys Ile Tyr Ser Ile
    1               5
<210> 25
   <211> 9
   <212> PRT
   <213> Homo sapiens
<400> 25
   Gly Tyr Tyr Asp Ala Gln Lys Leu Leu
    1               5
<210> 26
   <211> 9
   <212> PRT
   <213> Homo sapiens
<400> 26
   Thr Tyr Ser Val Ser Phe Asp Ser Leu
   1 5
<210> 27
   <211> 9
   <212> PRT
   <213> Homo sapiens
<400> 27
   Asn Tyr Ala Arg Thr Glu Asp Phe Phe
   1 5
<210> 28
   <211> 9
   <212> PRT
   <213> Homo sapiens
<400> 28
   Leu Tyr Ser Asp Pro Ala Asp Tyr Phe
   1 5
<210> 29
   <211> 9
   <212> PRT
   <213> Homo sapiens
<400> 29
   Leu Pro Ser Ile Pro Val His Pro Ile
   1 5
<210> 30
   <211> 9
   <212> PRT
   <213> Homo sapiens
<400> 30
   Ser Pro Ser Pro Glu Phe Ser Gly Met
   1 5
<210> 31
   <211> 9
   <212> PRT
   <213> Homo sapiens
<400> 31
   Val Leu Val His Pro Gln Trp Val Leu
   1 5
<210> 32
   <211> 9
   <212> PRT
   <213> Homo sapiens
<400> 32
   Lys Leu Gln Cys Val Asp Leu His Val
   1 5
<210> 33
   <211> 9
   <212> PRT
   <213> Homo sapiens
<400> 33
   Ala Leu Pro Glu Arg Pro Ser Leu Tyr
   1 5
<210> 34
   <211> 9
   <212> PRT
   <213> Homo sapiens
<400> 34
   Ile Val Gly Gly Trp Glu Cys Glu Lys
   1 5
<210> 35
   <211> 9
   <212> PRT
   <213> Homo sapiens
<400> 35
   Gln Val His Pro Gln Lys Val Thr Lys
   1 5
<210> 36
   <211> 9
   <212> PRT
   <213> Homo sapiens
<400> 36
   Val Val His Tyr Arg Lys Trp Ile Lys
   1 5
<210> 37
   <211> 9
   <212> PRT
   <213> Homo sapiens
<400> 37
   Cys Tyr Ala Ser Gly Trp Gly Ser Ile
   1 5